# EUROPÄISCHE PATENTSCHRIFT

(11) **EP 2 994 455 B1**
(45) Veröffentlichungstag und Bekanntmachung des Hinweises auf die Patenterteilung: **05.12.2018**
(21) Anmeldenummer: 14724366.1
(22) Anmeldetag: 06.05.2014
(51) Int. Cl.: C07D 233/58

(54) **VERFAHREN UND TECHNISCHER PROZESS ZUR KONTINUIERLICHEN SYNTHESE VON UNTERSCHIEDLICHEN IONISCHEN FLÜSSIGKEITEN**
METHOD AND TECHNICAL PROCESS FOR THE CONTINUOUS SYNTHESIS OF DIFFERENT IONIC LIQUIDS
MÉTHODE ET PROCÉDÉ TECHNIQUE POUR LA SYNTHÈSE CONTINUE DE LIQUIDES IONIQUES DIFFÉRENTS

(30) Priorität: 07.05.2013 DE 102013007733
(43) Veröffentlichungstag der Anmeldung: 16.03.2016
(73) Patentinhaber: Tuliport S.a.r.l., 1140 Luxemburg (LU)
(72) Erfinder: POMMERSHEIM, Rainer, 55116 Mainz (DE)
(74) Vertreter: Kruspig, Volkmar
(86) Internationale Anmeldenummer: PCT/EP2014/059150
(87) Internationale Veröffentlichungsnummer: WO 2014/180802

(56) Entgegenhaltungen:
- WO-A1-2012/123336
- WO-A2-2005/021484
- DE-A1-102008 032 595

## Beschreibung

Die Erfindung bezieht sich auf eine Methode zur Herstellung Ionischer Flüssigkeiten in einem kontinuierlichem Verfahren. Das Ganze ist so konzipiert, dass nahezu gleichzeitig unterschiedliche Ionische Flüssigkeiten erhalten werden. Hierzu bedient man sich einer chemischen Zwischenstufe, die nicht nur relativ einfach hergestellt, sondern auch ohne großen Aufwand in eine Vielzahl von Produkten umgewandelt wird. Die hier beschriebene Methode ermöglicht die Synthese von unterschiedlichen Ionischen Flüssigkeiten in hervorragenden Ausbeuten und Qualitäten in einem einheitlichen Produktionsprozess.
Unter dem Begriff der ionischen Flüssigkeiten (engl. "ionic liquids") versteht man Flüssigkeiten die ausschließlich aus Ionen aufgebaut sind. Es handelt sich dabei um geschmolzene Salze organischer Verbindungen oder eutektische Gemische von organischen und anorganischen Salzen.
Ionische Flüssigkeiten selbst besitzen eine Reihe von ausgezeichneten Eigenschaften: Sie sind nicht flüchtig (vernachlässigbarer Dampfdruck, wie Salze), nur schwer brennbar und thermisch stabil (je nach ausgewählter Flüssigkeit bis über 300°C). Die meisten ionischen Flüssigkeiten sind ungiftig.
Um eine Abgrenzung vom Begriff der klassischen Salzschmelze zu schaffen, welche hochschmelzend und stark korrosiv sind, wurde der Schmelzpunkt von ionischen Flüssigkeiten per Definition auf Temperaturen von unter 100°C festgelegt. Typisch für den Aufbau von Ionischen Flüssigkeiten sind große organische Kationen, genau genommen handelt es sich dabei um Onium - Ionen, welche oft durch Stickstoff oder Phosphorzentren und Alkylresten gebildet werden.
Diese Kationen können mit einer Vielzahl an organischen oder anorganischen Anionen kombiniert werden. Der modulare Aufbau solcher Flüssigkeiten erlaubt es, die physikalischen und chemischen Eigenschaften gezielt durch geeignete Zusammenstellung von Kationen und Anionen in einem weiten Bereich zu variieren. Primär wird durch die Wahl des Kations Stabilität und andere grundlegende physikalische Eigenschaften der Ionischen Flüssigkeit beeinflusst, während die Wahl des Anions die Chemie und Funktionalität bestimmt. Die Möglichkeit der schrittweisen Abstimmung relevanter chemischer als auch physikalischer Eigenschaften, ermöglicht die Entwicklung neuer ionischer Flüssigkeiten welche die Anforderungen konkreter Aufgaben zu 100% erfüllen.

Ionische Flüssigkeiten zeigen als alternative Lösungsmittel in chemischen und biokatalytischen Reaktionen interessante Eigenschaften: Ihre Nichtflüchtigkeit bietet verfahrenstechnische Vorteile. Zudem eröffnen ihre außergewöhnlichen Löslichkeitseigenschaften neue Möglichkeiten in chemischen Synthesen. Die ionischen Flüssigkeiten selbst können in den meisten Fällen nach der Verwendung leicht zurückgewonnen und erneut eingesetzt werden, was die Effizienz der chemischen Prozesse zusätzlich steigert.

Beispielhaft sind nachfolgend einige großtechnische Anwendungen Ionischer Flüssigkeiten aufgeführt:

### Chemische Industrie

Die BASF setzt in ihrem neuen BASIL (Biphasic Acid Scavenging utilizing lonic Liquids)-Verfahren weltweit erstmals Ionische Flüssigkeiten im industriellen Maßstab ein. Hier werden schädliche Säuren, die das Endprodukt zersetzen würden durch den Einsatz Ionischer Flüssigkeiten aus dem Prozess entfernt. Auf diese Weise konnte die Ausbeute des Verfahrens signifikant gegenüber der konventionellen Methode gesteigert werden.

Zellulose ist ein Grundstoff, der nicht nur in der Papier- und Faserindustrie eine zentrale Rolle spielt. Dank ihrer außergewöhnlichen Löslichkeitseigenschaften für Biopolymere eröffnen Ionische Flüssigkeiten wie beispielsweise Immodazolium Acetate völlig neue Möglichkeiten für neue Verfahren und Produkte. Durch deren Einsatz kann auf andere, toxische Lösungsmittel verzichtet werden.

In elektrochemischen Prozessen wie z.B. das Aluminiumplating bieten Ionische Flüssigkeiten die beispielsweise ein Immidazolium Kation und ein Chlorid-Anion enthalten als Elelktrolyt signifikante Vorteile gegenüber konventionellen Materialen.

Ionische Flüssigkeiten wie z.B. Immodazolium Sulfate eignen sich sehr gut als Antistatika für Kunststoffe.

### Petrochemie

Der durchschnittliche Schwefelgehalt im Rohöl ist in den letzen Jahrzehnten signifikant gestiegen. Dies nicht zuletzt deshalb weil immer mehr Lagerstätten niedriger Rohölqualität ausgebeutet werden. Heutige Diesel- und Benzinmotoren benötigen jedoch Kraftstoffe mit einem extrem niedrigen Schwefelgehalt. Auch kann Rohöl in den Raffinerien ab einem gewissen Schwefelgehalt nicht mehr verarbeitet werden. Deshalb werden bei der Aufbereitung des Rohöls große Anstrengungen unternommen, den Schwefelgehalt zu senken. Dies erfordert komplizierte chemische Verfahrensschritte teilweise mit einer hohen Umweltbelastung. Mit Hilfe Ionischer Flüssigkeiten wie z. B. Methyl-Imidazonium (MIM)-Derivaten, kann Schwefel durch einfaches Waschen aus dem Rohöl entfernt werden wie die Arbeiten mehrerer nationaler und internationaler Forschergruppen belegen.

### Elektrochemie

Aufgrund ihres ionischen Charakters verfügen Ionische Flüssigkeiten über ein großes Potenzial als Elektrolyte in elektrochemischen Speichern wie Batterien, Kondensatoren usw. Obwohl einige davon in so genannten Li-Ionenbatterien seit Jahren Verwendung finden, wird weltweit fieberhaft nach immer neuen Verbindungen und Herstellungsmethoden für Ionische Flüssigkeiten für diesen Anwendungszweck gesucht.

### Photovoltaik

So genannte DSSC-Photovoltaik-Module sind eine neue Generation von Zellen, von denen man sich sehr viel verspricht. Sie funktionieren nach einem Prinzip ähnlich der pflanzlichen Photosynthese und können auch bei schlechtem oder diffusem Licht noch relativ hohe Energieausbeuten liefern. Um den Ladungsaustausch innerhalb der DSSC-Zellen zu ermöglichen, sind spezielle Elektrolyte mit ganz besonderen Eigenschaften erforderlich. Ionische Flüssigkeiten erfüllen diese Anforderungen. Deshalb ist die Entwicklung der DSSC-Zellen eng mit der Ionischer Flüssigkeiten verknüpft.

Vor diesem Hintergrund ist es nicht verwunderlich, dass sowohl zahlreiche Synthesemöglichkeiten als auch immer mehr Anwendungen für Ionische Flüssigkeiten in der Literatur der letzten Jahre beschrieben sind. Beispielhaft sind nachfolgend einige davon zusammengefasst:
In der Offenlegungsschrift DE 10 2005 025 531A1 werden z.B. unterschiedliche Ionische Flüssigkeiten niedriger Viskosität und hoher elektrochemischer Stabilität beschreiben, die hauptsächlich für elektrochemische Anwendungen gedacht sind. Auch werden mehrere Synthesewege aufgezeigt, wie diese Verbindungen im Labor hergestellt werden können.

Mit der Herstellung im Labormaßstab von Ionischen Flüssigkeiten mit Alkylsulfaten oder funktionalisierten Alkylsulfaten als Anion, befasst sich die Patentanmeldung DE 103 19 465 A1**.** Diese Verbindungen sind als halogenfreie Lösungsmittel, Extraktionsmittel und Wärmeträger von erheblicher technischer Bedeutung.

Ein Laborverfahren zur Herstellung von Ionischen Flüssigkeiten mit halogenhaltigem Anion hat die Patentanmeldung EP 1 182 196 A1 zum Gegenstand.

Alkylammoniomsalze als Kation einer Ionischen Flüssigkeit und deren Herstellungsverfahren sind in der Anmeldung GB 2 444 614 A1 beschrieben.

Ionische Flüssigkeiten mit Alkylsulfaten als Anionen, sowie eine Lobormethode zu deren Herstellung sind Gegenstand der Anmeldung US 2008/033 178 A1**.**

Ein großes Problem bei der Herstellung Ionischer Flüssigkeiten in größeren Mengen ist die Kontrolle der Temperatur während des Reaktionsprozesses. Um die Ausgangsstoffe zur Reaktion zu bringen, muss dem System erst einmal Wärme zugeführt werden. Ist die Reaktion aber dann im Gange, verläuft diese stark exotherm, war eine effiziente Abführung der entstehenden Wärme aus dem System erfordert.

Die Anmeldung DE 10 2008 032 595 A1 befasst sich eingehend mit dieser Problematik und beschreibt ein technisches Verfahren, bei dem sowohl die erforderliche Aktivierungswärme als auch die entstehende Reaktionswärme durch die Verwendung eines geeigneten Lösungsmittels kontrolliert wird. Mit dem in DE 10 2008 032 595 A1 beschriebenen Prozess ist die nahezu gleichzeitige Herstellung mehrerer unterschiedlicher ionischer Flüssigkeiten nicht möglich.

All diese Literaturstellen zeigen, wie vielfältig die lonenzusammenstellungen und die Synthesemöglichkeiten für Ionische Flüssigkeiten sind. Die beschriebenen Methoden sind im Labormaßstab praktikabel und in Einzelfällen auch für eine technisch Produktion geeignet.

Sie stoßen jedoch an ihre Grenzen, wenn es darum geht mahrere Sustanzen im technischen Maßstab in einem einheitlichen Prozess herzustellen.

Ein Betrieb, der unterschiedliche ionische Flüssigkeiten anbieten wollte, müsste entweder unterschiedliche Anlagen betreiben oder bestehende Anlagen laufend umrüsten. Dies ist nicht aufwändig sondern in vielen Fällen auch nicht wirtschaftlich.

WO 2005/021484 A2 betrifft ein Verfahren zur Herstellung ionischer Flüssigkeiten, ionischer Feststoffe oder Gemische derselben aus kationischen Synthesemodulen [Q+] n [Yn-] und anionischen Synthese-modulen, wobei [Q+] ein quaterniertes Ammonium- [R1R2R3R4N+], Phosphonium- [R1R2R3R4P+] oder Sulfonium- [R1R2R3S+] Kation oder ein analoger quaternierter Stickstoff-, Phosphor-oder Schwefel-Heteroaromat ist.
In WO 2012/123336 A1 wird ein Verfahren zur Herstellung von ionischen Flüssigkeiten durch Anionenaustausch offenbart. Dabei kommen Lösungsmittel zum Einsatz, die gemäß der dortigen Lehre ausführlich erläutert werden. Es handelt sich primär um Wasser oder zumindest zum Teil mit Wasser homogen mischbare, organische Verbindungen. Der ausschließliche Zweck ist, die an der Reaktion beteiligten Stoffe in Lösung zu bringen.

Darüber hinaus offenbart WO 2012/123336 A1 Methoden und Reaktionen, wie man an Verbindungen des Typs K+Y- einen lonenaustausch vornehmen kann, um ionische Flüssigkeiten zu erhalten.

Ausgehend von dieser Sachlage, liegt der vorliegenden Erfindung die Aufgabe zugrunde, eine Methode sowie einen technischen Prozess zu beschreiben mit dem in einem einheitlichen Produktionsablauf unterschiedliche ionische Flüssigkeiten nahezu zeitgleich hergestellt werden können.

Die Grundidee des Verfahrens besteht demnach darin, in einem kontinuierlichen Verfahren, eine Zwischenstufe zu synthetisieren, die mit einfachen, konventionellen Mitteln die unterschiedliche Endprodukte, also ionische Flüssigkeiten überführt werden kann.

Eine solche geeignete Zwischenstufe könnten so genannte Immidazolium basierte Carboxylate sein. Verfahren zu deren Herstellung sind literaturbekannt. So wird beispielsweise in Green Process Synth 1 (2012): 261-267 ein Laborprozess beschrieben, bei dem N-Methyimidazol mit Dimethylcarbonat alkyliert wird. Geht man von anderen Alkylierungsreagenzien aus, wie in Chemical Engineering Journal 163 (2010) 429-437 beschrieben erhält man beispielsweise die entsprechenden Halogenide oder Sulfate des Methylimidazols.

Derlei Zwischenstufen können anschließend durch das Versetzen mit Säuren wie z.B. Essigsäure z.B in das entsprechende Immidazolium Acetat, durch die Reaktion mit Salzsäure in das Immidazolium Chlorid oder mit Salpetersäure in das entsprechende Nitrat verwandelt werden, also unterschiedliche Ionische Flüssigkeiten, die auf dem Immidazolium Kation basieren.

Da immer von der gleichen oder von synthesetechnisch vergleichbaren Zwischenstufen ausgegangen wird, kann man die unterschiedlichen Ionischen Flüssigkeiten innerhalb des selben technischen Prozesse herstellen.

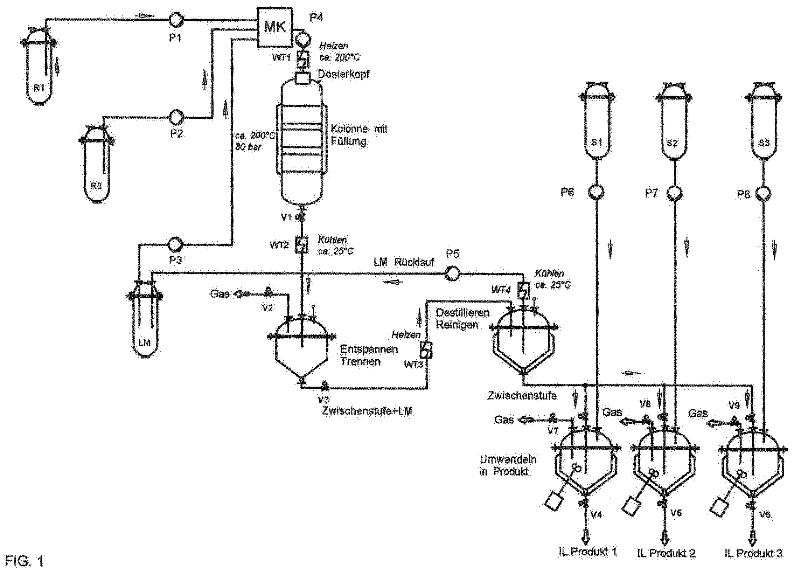

Beispiele für einen möglichen technischen Prozess, zur Herstellung von Ionischen Flüssigkeiten wie er in der vorliegenden Erfindung beschrieben ist, werden nachfolgend anhand des Schemas aus Fig. 1 gezeigt:

### Ausführungsbeispiel 1:

Bei dem Prozess aus **Fig. 1** werden die Ausgangssubstanzen **R1** (z.B. Dimethylcarbonat) und **R2** (z.B. Methylimidazol), sowie ein Lösungsmittel (z.B. Methanol) in entsprechenden Behältern vorgelegt und von dort über die Pumpen [**P1, P2** und **P3**] zu einer Mischkammer [**MK**] transportiert. Das Lösemittel hat unter anderem hat die Aufgabe, die Reaktionstemperatur in engen Grenzen zu halten. Aus der Mischkammer wird das Gemisch über die Pumpe [**P4**] am Kopf eines kontinuierlich betriebenen Reaktors unter Druck (z.B. p= 80 bar) aufgegeben. Zuvor kann das Gemisch vorerwärmt werden. Die Aufgabe kann dabei durch eine geeignete Vorrichtung, z.B. eine einzelne Düse oder durch Düsenarrays in Form von Tropfen, von fließender Flüssigkeit, oder durch Versprühen erfolgen.

Der kontinuierlich betriebene Reaktor wird in geeigneter Weise, z.B. von Außen oder durch Elemente von Innen, beheizt oder gekühlt, um die Reaktionstemperatur einstellen zu können. Der Reaktor kann zusätzlich Einbauten enthalten, die eine enge Verweilzeitverteilung ermöglichen. Im Reaktor befindet sich eine Füllung die entweder aus gebräuchlichen Füllmaterialien wie Raschig-Ringen oder Ähnlichem besteht. Sie kann aber auch Substanzen enthalten, die eine katalytische Wirkung entfalten, wie beispielsweise Metalloxide. Die Temperatur im Reaktor beträgt ca. 200° C, der Druck liegt bei ca. 80 bar.

Die Ausgangssubstanzen reagieren bei der Passage durch den Reaktor. Dabei lösen sich entweder nicht umgesetzte Ausgangssubstanzen oder die Ionische Flüssigkeit in dem eingesetzten Lösemittel. Nach Verlassen des Reaktors über das Ventil [**V1**] wird das entstandene Zwischenprodukt (z.B. das Methylimmiodazolimcarboxylat) zusammen mit dem Lösungsmittel im Wärmetrauscher [**WT2**] auf Raumtemperatur gekühlt und in die Trenneinheit geleitet. Dort findet ein Entspannen statt, das bei der Reaktion entstandene Gas (z.B. CO₂) wird entfernt.

Aus der Trenneinheit wird das Gemisch einer Destillation zugeführt, wo das Zwischenprodukt (Zwischenstufe) von dem Lösungsmittel getrennt wird. Das Lösungsmittel wird über die Pumpe [**P6**] wieder zurück geführt. Zuvor kann das Gemisch über den Wärmetauscher [**WT3**] vorerwärmt werden. WT3 kann mit WT2 so gekoppelt sein, dass eine Wärmerückgewinnung erreicht wird.

Die so erhaltene Zwischenstufe reagiert anschließend zu dem gewünschten Endprodukt. Dies erfolgt in einem oder wie in Fig. 1 dargestellt mehreren Reaktionsgefäßen. In diesen Behältern wird die Zwischenstufe mit einer geeigneten Säure versetzt, wodurch CO₂ und das gewünschte Endprodukt aus der Zwischenstufe entsteht. Unterschiedliche Säuren führen zu unterschiedlichen Produkten. So entsteht z.B. bei der Zugabe von Essigsäure das Methylimmidazoliumacetat, mit Salzsäure, das entsprechende Chlorid, mit Salpetersäure das entsprechende Nitrat usw. Die Säuren (S1, S2, S3 usw.) werden über die Dosierpumpen [**P6**], [**P7**], [**P8**] dem jeweiligen Reaktor zugeführt. Über die Ventile [**V4**], [**V5**] und [**V6**] werden die entstanden Ionische Flüssigkeiten (IL Produkt 1, IL Produkt 2, IL Podukt 3 usw.) einer nachfolgenden Aufarbeitung bzw. Reinigung zugeführt.

### Ausführungsbeispiel 2:

Der Prozess aus **Fig. 1** kann auch dazu verwendet werden einzelne, spezifische Ionische direkt, also nicht über eine Zwischenstufe herzustellen. Dafür müssen andere Ausgangsstoffe als in dem Beispiel 1 eingesetzt werden.
Die Ausgangssubstanzen **R1** (z.B. Diethylsulfat) und **R2** (z.B. Methylimidazol) werden über die Pumpen [**P1** und **P2**] zu einer Mischkammer [**MK**] transportiert. Die Mischkammer kann über eine Kühl- oder Heizvorrichtung auf die Ausgangstemperatur gebracht werden. Diesem Gemisch wird eine geeignete Menge eines Lösemittels (z.B. Toluol, Ethylacetat suw.) über die Pumpe [**P3**] kontinuierlich zugeführt. Das Lösemittel hat die Aufgabe, die Reaktionstemperatur in engen Grenzen zu halten. Dabei sollten entweder die zu bildenden Ionische Flüssigkeit oder nicht umgesetzte Ausgangssubstanzen in dem gewählten Lösemittel löslich sein. Die Komponenten R1, R2 und das Lösemittel LM werden über die Pumpe [**P4**] am Kopf eines kontinuierlich betriebenen Reaktors aufgegeben. Die Aufgabe kann dabei durch eine geeignete Vorrichtung z.B. eine einzelne Düse oder durch Düsenarrays in Form von Tropfen, von fließender Flüssigkeit, oder durch Versprühen erfolgen.

Der kontinuierlich betriebene Reaktor wird in geeigneter Weise, z.B. von Außen oder durch Elemente von Innen, beheizt oder gekühlt, um die Reaktionstemperatur einstellen zu können. Der Reaktor kann zusätzlich Einbauten enthalten, die eine enge Verweilzeitverteilung ermöglichen, oder eine katalytische Wirkung entfalten. Je nach Erfordernis kann im Reaktor zusätzlich ein Temperaturgradient eingestellt werden.

Die Ausgangssubstanzen reagieren bei der Passage durch den Reaktor. Dabei lösen sich entweder nicht umgesetzte Ausgangssubstanzen oder die Ionische Flüssigkeit in dem eingesetzten Lösemittel. Nach Verlassen des Reaktors über das Ventil [**V1**] werden die entstandenen flüssigen Phasen in der Trenneinheit getrennt. Der überwiegende Teil des Lösungsmittels, das eine zweite Phase mit dem Produkt bildet wird z.B. über den

Gasanschluss und über eine zusätzlich zu installierende Pumpe in den Lösungsmittelbehälter zurückgeführt. Über [**V3**] wird in diesem Fall bereits das Endprodukt der Destillation zugeführt, wo es von den Lösungsmitteltesten gesäubert wird. Am Ausgang der Destilliereinheit erhält man somit bereits das Endprodukt. Verwendet man die Ausgangssubstanzen aus diesem Beispiel ist es das Methylimmidazoliumdiethylsulfat.

## Patentansprüche

1. Verfahren zur Synthese von Ionischen Flüssigkeiten, bei dem in einem einheitlichen Produktionsablauf unterschiedliche Ionische Flüssigkeiten hergestellt werden, **dadurch gekennzeichnet,**
**dass** die Synthese über eine chemische Zwischenstufe verläuft, die mit konventionellen Mitteln in unterschiedliche Endprodukte umgewandelt wird, wobei ein zugesetztes Lösemittel den Wärmetransport übernimmt und die Reaktionstemperatur durch dessen Verdampfungstemperatur einstellt und somit der Temperaturkontrolle dient.

2. Verfahren zur Synthese von Ionischen Flüssigkeiten nach Anspruch 1,
**dadurch gekennzeichnet,**
**dass** die chemische Zwischenstufe ein Carboxylat, ein Carbonat oder eine vergleichbare Verbindung ist.

3. Verfahren zur Synthese von Ionischen Flüssigkeiten nach Anspruch 1 oder 2,
**dadurch gekennzeichnet**,
die chemische Zwischenstufe durch Zugabe einer organischen oder anorganischen Säure in das Endprodukt umgewandelt wird.

4. Verfahren zur Synthese von Ionischen Flüssigkeiten nach Anspruch 1 bis 3,
**dadurch gekennzeichnet,**
**dass** der Reaktor Einbauten oder eine Füllung besitzt, die auch eine katalytische Wirkung entfalteten.

5. Verfahren zur Synthese von Ionischen Flüssigkeiten nach Anspruch 1 bis 4,
**dadurch gekennzeichnet,**
**dass** die Einbauten oder die Füllung im Reaktor aus Elementen besteht, die aus Metall, Glas, Kohlenstoff oder einem Metalloxyd bestehen oder aber wie sie in Destillierkolonnen in der chemischen Industrie zum Einsatz kommen.

6. Verfahren zur Synthese von Ionischen Flüssigkeiten nach Anspruch 1 bis 5,
**dadurch gekennzeichnet,**
**dass** als Ausgangssubstanzen offenkettige oder cyclische Verbindungen verwendet werden, die bei der Reaktion mit einem geeigneten Alkylierungsmittel Ionische Flüssigkeiten des Typs A⁺B⁻ ergeben, wobei A ein Stoff aus der Klasse der Amine oder Imine ist und B ein Stoff aus der Klasse der Halogenalkane oder der substituierten Halogenalkane ist.

7. Verfahren nach Anspruch 6,
**dadurch gekennzeichnet, dass**
A ein Pyridin oder Piperidin oder Imidazol ist.

8. Verfahren nach Anspruch 6 oder 7,
**dadurch gekennzeichnet, dass**
B ein Diethylsulfat oder Dimethylcarbonat ist.

## Claims

1. A method for synthesis of ionic liquids, in which, in a uniform production sequence, different ionic liquids are produced, **characterized in that** the synthesis runs via a chemical intermediate stage, which are converted using conventional means into different final products,
wherein an added solvent assumes the heat transport, and sets the reaction temperature by way of its vaporization temperature, and thus is used for temperature control.

2. The method for synthesis of ionic liquids according to Claim 1, **characterized in that** the chemical intermediate stage is a carboxylate, a carbonate, or a comparable compound.

3. The method for synthesis of ionic liquids according to Claim 1 or 2, **characterized in that** the chemical intermediate stage is converted into the final product by adding an organic or inorganic acid.

4. The method for synthesis of ionic liquids according to Claims 1 to 3, **characterized in that** the reactor has fittings or a filler which also unfolds a catalytic effect.

5. The method for synthesis of ionic liquids according to Claims 1 to 4, **characterized in that** the fittings or the filler in the reactor consist of elements which consist of metal, glass, carbon, or a metal oxide, or as are used in distillation columns in the chemical industry.

6. The method for synthesis of ionic liquids according to Claims 1 to 5, **characterized in that** open-chain or cyclic compounds are used as the starting substances, which results upon the reaction with a suitable alkylation agent in ionic liquids of the type A⁺B⁻, wherein A is a material from the class of amines or imines, and B is a material from the class of halogen alkanes or substituted halogen alkanes.

7. The method according to Claim 6, **characterized in that** A is a pyridine or piperidine or imidazole.

8. The method according to Claim 6 or 7, **characterized in that** B is a diethyl sulfate or dimethyl carbonate.

## Revendications

1. Procédé de synthèse de liquides ioniques, dans lequel différents liquides ioniques sont produits dans un déroulement de production unitaire,
**caractérisé en ce que**
la synthèse passe par un stade chimique intermédiaire qui est transformé en différents produits finaux par des moyens classiques,
un solvant ajouté assure le transfert thermique et règle la température de réaction par sa température d'évaporation et sert donc au contrôle de la température.

2. Procédé de synthèse de liquides ioniques selon la revendication 1,
**caractérisé en ce que**
le stade chimique intermédiaire est un carboxylate, un carbonate ou un composé comparable.

3. Procédé de synthèse de liquides ioniques selon la revendication 1 ou 2,
**caractérisé en ce que**
le stade chimique intermédiaire est transformé en le produit final par ajout d'un acide organique ou inorganique.

4. Procédé de synthèse de liquides ioniques selon la revendication 1 à 3,
**caractérisé en ce que**
le réacteur possède des aménagements ou un remplissage qui développent également un effet catalytique.

5. Procédé de synthèse de liquides ioniques selon la revendication 1 à 4,
**caractérisé en ce que**
les aménagements ou le remplissage dans le réacteur sont constitués par des éléments qui sont constitués en métal, en verre, en carbone ou en un oxyde de métal, ou bien par des éléments tels qu'ils sont utilisés dans des colonnes de distillation dans l'industrie chimique.

6. Procédé de synthèse de liquides ioniques selon la revendication 1 à 5,
**caractérisé en ce que**
en tant que substances de départ, on utilise des composés à chaîne ouverte ou cycliques qui, lors de la réaction avec un agent alkylant approprié, donnent des liquides ioniques du type A⁺B⁻, A étant une substance de la classe des amines ou imines, et B étant une substance de la classe des halogénoalcanes ou des halogénoalcanes substitués.

7. Procédé selon la revendication 6,
**caractérisé en ce que**
A est une pyridine ou pipéridine ou un imidazole.

8. Procédé selon la revendication 6 ou 7,
**caractérisé en ce que**
B est un sulfate de diéthyle ou un carbonate de diméthyle.
